# EUROPEAN PATENT SPECIFICATION

(11) **EP 3 548 065 B1**
(45) Date of publication and mention of the grant of the patent: **09.11.2022**
(21) Application number: 17816576.7
(22) Date of filing: 30.11.2017
(51) Int. Cl.: A61K 38/45, A61K 48/00, C12N 15/861, A61P 27/02

(54) **PHARMACEUTICAL COMPOSITIONS FOR THE TREATMENT OF RETINAL DEGENERATIVE DISEASES**
PHARMAZEUTISCHE ZUSAMMENSETZUNGEN ZUR BEHANDLUNG VON DEGENERATIVEN NETZHAUTERKRANKUNGEN
COMPOSITIONS PHARMACEUTIQUES POUR LE TRAITEMENT DE DÉGÉNÉRESCENCES RÉTINIENNES

(30) Priority: 01.12.2016 EP 16306592
(43) Date of publication of application: 09.10.2019
(73) Proprietor: INSERM - Institut National de la Santé et de la Recherche Médicale, 75013 Paris (FR); SORBONNE UNIVERSITE, 75006 Paris (FR); Centre National de la Recherche Scientifique (CNRS), 75016 Paris (FR)
(72) Inventor: LEVEILLARD, Thierry, 75012 Paris (FR); MILLET-PUEL, Géraldine, 75012 Paris (FR); BOUAZIZ, Alexandra Lyor, 75012 Paris (FR)
(74) Representative: Inserm Transfert
(86) International application number: PCT/EP2017/080956
(87) International publication number: WO 2018/100054

(56) References cited:
- WO-A1-2014/207190
- WO-A2-2004/007673
- AÏT-ALI NAJATE ET AL: "Rod-Derived Cone Viability Factor Promotes Cone Survival by Stimulating Aerobic Glycolysis", CELL, vol. 161, no. 4, 7 May 2015 (2015-05-07), pages 817-832, XP029224282, ISSN: 0092-8674, DOI: 10.1016/J.CELL.2015.03.023
- HEINE-SUENER D ET AL: "SEQUENCE AND STRUCTURE OF THE HUMAN 6-PHOSPHOFRUCTO-2-KINASE/FRUCTOSE-2,6-BISP HOSPHATASE HEART ISOFORM GENE", EUROPEAN JOURNAL OF BIOCHEMISTRY, WILEY-BLACKWELL PUBLISHING LTD, GB, vol. 254, January 1998 (1998-01), pages 103-110, XP002906607, ISSN: 0014-2956, DOI: 10.1046/J.1432-1327.1998.2540103.X
- KIM S G ET AL: "A Direct Substrate-Substrate Interaction Found in the Kinase Domain of the Bifunctional Enzyme, 6-Phosphofructo-2-kinase/Fructose-2,6-bisp hosphatase", JOURNAL OF MOLECULAR BIOLOGY, ACADEMIC PRESS, UNITED KINGDOM, vol. 370, no. 1, 29 June 2007 (2007-06-29) , pages 14-26, XP025323302, ISSN: 0022-2836, DOI: 10.1016/J.JMB.2007.03.038 [retrieved on 2007-05-30]

## Description

### FIELD:

The present disclosure is in the field of medicine, in particular ophthalmology.

### BACKGROUND:

RdCVF, a truncated thioredoxin-like protein lacking thioloxidoreductase activity, was identified by high content screening of a mouse retinal cDNA library on cone-enriched cultures from chicken embryos (Léveillard et al., 2004). RdCVF is an alternative splice variant of the nucleoredoxin-like 1 *(Nxnl1)* gene, whose other splice product is RdCVFL, an active thioredoxin that protects its binding partner, the microtubule associated protein TAU, from oxidation and aggregation (Elachouri et al., 2015 and Fridlich et al., 2009). RdCVF, but not RdCVFL, protects cone function in several genetically distinct models of RP, targeting the most debilitating step in that untreatable disease (Byrne et al., 2015, Léveillard et al., 2004 and Yang et al., 2009). Because the secondary loss of cones in retinitis pigmentosa (RP) leads to blindness, the administration of RdCVF represent a promising therapy for this untreatable retinal degenerative disease. Recently the mechanism underlying the protective role of RdCVF in RP was investigated. RdCVF acts through binding to Basigin-1 (BSG1), a transmembrane protein expressed specifically by photoreceptors. BSG1 binds to the glucose transporter GLUT1, resulting in increased glucose entry into cones (Aït-Ali et al. 2015). Identification of the remaining key actors of said mechanism would thus lead to the conception new therapy of the retinal degenerative disease.

### SUMMARY OF THE INVENTION:

The present invention is defined by the claims.

In particular, the present invention relates to a polynucleotide encoding for the PFKFB2 kinase domain for use in the treatment of a retinal degenerative disease in a subject in need thereof.

The following detailed description, figures and example do not fall under the scope of the present invention and are present for understanding and illustration purposes only. In particular, the references to methods of treatment in this description are to be interpreted as references to the compounds, pharmaceutical compositions and medicaments of the present invention for use in a method for the treatment of the human body by therapy.

### DETAILED DESCRIPTION:

The inventors identified a new key actor of the mechanism underlying the protective role of RdCVF: 6-phosphofructo-2-kinase/fructose-2,6-bisphosphatase 2 (PFKFB2). This identification paves the way of new strategies for the treatment of retinal degenerative diseases.

Accordingly, the present disclosure relates to a method of treating a retinal degenerative disease in a subject in need thereof comprising administering to the subject a therapeutically effective amount of a polynucleotide encoding for the PFKFB2 kinase domain.

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein the term "retinal degenerative diseases" encompasses all retinal diseases associated with cone degeneration. The method of the present disclosure is thus particularly suitable for preventing cone degeneration. In particular, the retinal degenerative disease is cone dystrophy. As used herein the term "cone dystrophy" has its general meaning in the art and refers to an ocular disorder characterized by the loss of cone cells, the photoreceptors responsible for both central and color vision. The most common symptoms of cone dystrophy are vision loss (age of onset ranging from the late teens to the sixties), sensitivity to bright lights, and poor color vision. Thus the present disclosure is thus suitable for preventing vision loss of a patient suffering from a retinal degenerative disease. Particular examples of retinal degenerative diseases include retinitis pigmentosa (RP), Leber congenital amaurosis (LCA), age-related macular degeneration (AMD), recessive RP, dominant RP, X-linked RP, incomplete X-linked RP, dominant, dominant LCA, recessive ataxia, posterior column with RP, recessive RP with para-arteriolar preservation of the RPE, RP 12, Usher syndrome, dominant retinitis pigmentosa with sensorineural deafness, recessive retinitis punctata albescens, recessive Alstrδm syndrome, recessive Bardet-Biedl syndrome, dominant spinocerebellar ataxia w/ macular dystrophy or retinal degeneration, Recessive abetalipoproteinemia, recessive retinitis pigmentosa with macular degeneration, recessive Refsum disease adult form, recessive Refsum disease infantile form, recessive enhanced S-cone syndrome, RP with mental retardation, RP with myopathy, recessive Newfoundland rod-cone dystrophy, RetRP sinpigmento, sector RP, regional RP, Senior-Loken syndrome, Joubert syndrome, Stargardt disease juvenile, Stargardt disease late onset, dominant macular dystrophy Stargardt type, dominant Stargardt-like macular dystrophy, recessive macular dystrophy, recessive fundus flavimaculatus, recessive cone-rod dystrophy, X- linked progressive cone-rod dystrophy, dominant cone-rod dystrophy, cone-rod dystrophy; de Grouchy syndrome, dominant cone dystrophy, X-linked cone dystrophy, recessive cone dystrophy, recessive cone dystrophy with supernormal rod electroretinogram, X-linked atrophic macular dystrophy, X-linked retinoschisis, dominant macular dystrophy, dominant radial, macular drusen, dominant macular dystrophy, bull's-eye, dominant macular dystrophy butterfly-shaped, dominant adult vitelliform macular dystrophy, dominant macular dystrophy North Carolina type, dominant retinal-cone dystrophy 1, dominant macular dystrophy cystoid, dominant macular dystrophy, atypical vitelliform, foveomacular atrophy, dominant macular dystrophy Best type, dominant macular dystrophy North Carolina-like with progressive, recessive macular dystrophy juvenile with hypotrichosis, recessive foveal hypoplasia and anterior segment dysgenesis, recessive delayed cone adaptation, macular dystrophy in blue cone monochromacy, macular pattern dystrophy with type II diabetes and deafness, Flecked retina of Kandori, pattern dystrophy, dominant Stickler syndrome, dominant Marshall syndrome, dominant vitreoretinal degeneration, dominant familial exudative vitreoretinopathy, dominant vitreoretinochoroidopathy; dominant neovascular inflammatory vitreoretinopathy, Goldmann-Favre syndrome, recessive achromatopsia, dominant tritanopia, recessive rod monochromacy, congenital red-green deficiency, deuteranopia, protanopia, deuteranomaly, protanomaly, recessive Oguchi disease, dominant macular dystrophy late onset, recessive gyrate atrophy, dominant atrophia areata, dominant central areolar choroidal dystrophy, X-linked choroideremia, choroidal atrophy, central areolar, central, peripapillary, dominant progressive bifocal chorioretinal atrophy, progresive bifocal choroioretinal atrophy, dominant Doyne honeycomb retinal degeneration (Malattia Leventinese), amelogenesis imperfecta, recessive Bietti crystalline corneoretinal dystrophy, dominant hereditary vascular retinopathy with Raynaud phenomenon and migraine, dominant Wagner disease and erosive vitreoretinopathy, recessive microphthalmos and retinal disease syndrome; recessive nanophthalmos, recessive retardation, spasticity and retinal degeneration, recessive Bothnia dystrophy, recessive pseudoxanthoma elasticum, dominant pseudoxanthoma elasticum; recessive Batten disease (ceroid-lipofuscinosis), juvenile, dominant Alagille syndrome, McKusick- Kaufman syndrome, hypoprebetalipoproteinemia, acanthocytosis, palladial degeneration; Recessive Hallervorden-Spatz syndrome; dominant Sorsby's fundus dystrophy, Oregon eye disease, Kearns-Sayre syndrome, RP with developmental and neurological abnormalities, Basseb Korenzweig Syndrome, Hurler disease, Sanfilippo disease, Scieie disease, melanoma associated retinopathy, Sheen retinal dystrophy, Duchenne macular dystrophy, Becker macular dystrophy, Birdshot Retinochoroidopathy, multiple evanescent white-dot syndrome, acute zonal occult outer retinopathy, retinal vein occlusion, retinal artery occlusion, diabetic retinopathy, retinal toxicity, retinal injury, retinal traumata and retinal laser lesions, and Fundus Albipunctata, retinal detachment, diabetic retinopathy, retinopathy of prematurity.

As used herein the term "PFKFB2" has its general meaning in the art and refers to the 6-phosphofructo-2-kinase/fructose-2,6-biphosphatase 2 encoded by the PFKFB2 gene (Gene ID : 5208). PFKFB2 is involved in both the synthesis and degradation of fructose-2,6-bisphosphate, a regulatory molecule that controls glycolysis in eukaryotes and has a 6-phosphofructo-2-kinase activity that catalyzes the synthesis of fructose-2,6-bisphosphate, and a fructose-2,6-biphosphatase activity that catalyzes the degradation of fructose-2,6-bisphosphate. An exemplary amino acid sequence of PFKFB2 is reported at NCBI accession number NP_006203.2 and is represented by SEQ ID NO:1.

SEQ ID NO:1
MSGASSSEQN NNSYETKTPN LRMSEKKCSW ASYMTNSPTL IVMIGLPARG
KTYVSKKLTR YLNWIGVPTK VFNLGVYRRE AVKSYKSYDF FRHDNEEAMK
IRKQCALVAL EDVKAYLTEE NGQIAVFDAT NTTRERRDMI LNFAEQNSFK
VFFVESVCDD PDVIAANILE VKVSSPDYPE RNRENVMEDF LKRIECYKVT

YRPLDPDNYD KDLSFIKVIN VGQRFLVNRV QDYIQSKIVY YLMNIHVQPR
TIYLCRHGES EFNLLGKIGG DSGLSVRGKQ FAQALRKFLE EQEITDLKVW
TSQLKRTIQT AESLGVPYEQ WKILNEIDAG VCEEMTYAEI EKRYPEEFAL
RDQEKYLYRY PGGESYQDLV QRLEPVIMEL ERQGNVLVIS HQAVMRCLLA
YFLDKGADEL PYLRCPLHTI FKLTPVAYGC KVETIKLNVE AVNTHRDKPT
NNFPKNQTPV RMRRNSFTPL SSSNTIRRPR NYSVGSRPLK PLSPLRAQDM QEGAD

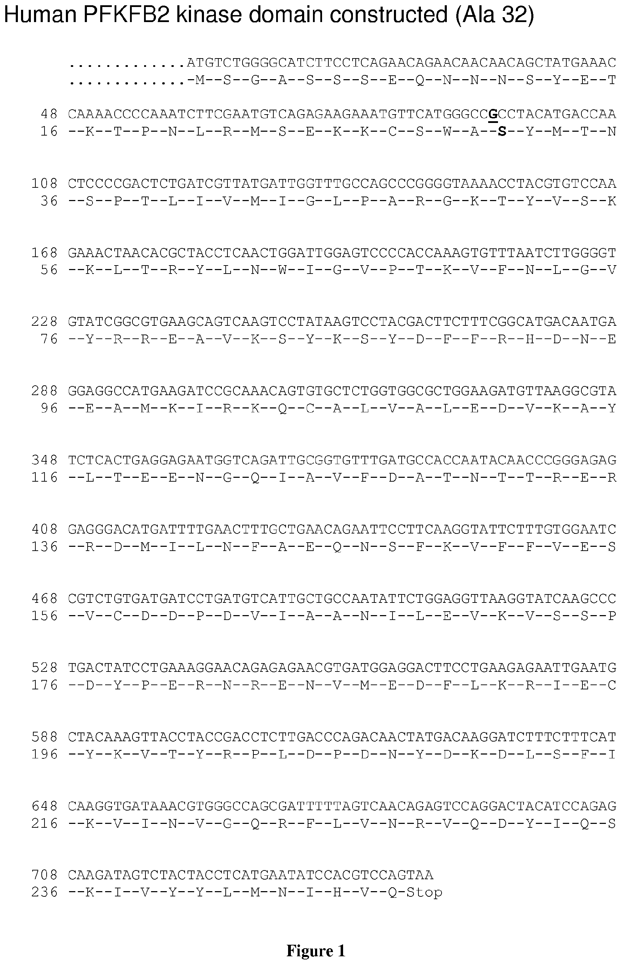

As used herein, the term "PFKFB2 kinase domain" refers to the domain responsible for the kinase activity of PFKFB2. Typically, said domain corresponds to the amino acid residue at position 1 to the amino acid residue at position 247 in SEQ ID NO:1 and is represented by SEQ ID NO: 2 (see Figure 1).

SEQ ID NO:2
MSGASSSEQNNNSYETKTPNLRMSEKKCSWASYMTNSPTLIVMIGLPARGKT
YVSKKLTRYLNWIGVPTKVFNLGVYRREAVKSYKSYDFFRHDNEEAMKIRKQ
CALVALEDVKAYLTEENGQIAVFDATNTTRERRDMILNFAEQNSFKVFFVESV
CDDPDVIAANILEVKVSSPDYPERNRENVMEDFLKRIECYKVTYRPLDPDNYD
KDLSFIKVINVGQRFLVNRVQDYIQSKIVYYLMNIHVQ

In particular, the polynucleotide of the present disclosure encodes for an amino acid sequence having at least 80% of identity with SEQ ID NO:2.

According to the present disclosure a first amino acid sequence having at least 80% of identity with a second amino acid sequence means that the first sequence has 80; 81; 82; 83; 84; 85; 86; 87; 88; 89; 90; 91; 92; 93; 94; 95; 96; 97; 98; 99 or 100% of identity with the second amino acid sequence. Sequence identity is frequently measured in terms of percentage identity (or similarity or homology); the higher the percentage, the more similar are the two sequences. Methods of alignment of sequences for comparison are well known in the art. Various programs and alignment algorithms are described in: Smith and Waterman, Adv. Appl. Math., 2:482, 1981; Needleman and Wunsch, J. Mol. Biol., 48:443, 1970; Pearson and Lipman, Proc. Natl. Acad. Sci. U.S.A., 85:2444, 1988; Higgins and Sharp, Gene, 73:237-244, 1988; Higgins and Sharp, CABIOS, 5:151-153, 1989; Corpet et al. Nuc. Acids Res., 16:10881-10890, 1988; Huang et al., Comp. Appls Biosci., 8:155-165, 1992; and Pearson et al., Meth. Mol. Biol., 24:307-31, 1994). Altschul et al., Nat. Genet., 6:119-129, 1994, presents a detailed consideration of sequence alignment methods and homology calculations. By way of example, the alignment tools ALIGN (Myers and Miller, CABIOS 4:11-17, 1989) or LFASTA (Pearson and Lipman, 1988) may be used to perform sequence comparisons (Internet Program^{®} 1996, W. R. Pearson and the University of Virginia, fasta20u63 version 2.0u63, release date December 1996). ALIGN compares entire sequences against one another, while LFASTA compares regions of local similarity. These alignment tools and their respective tutorials are available on the Internet at the NCSA Website, for instance. Alternatively, for comparisons of amino acid sequences of greater than about 30 amino acids, the Blast 2 sequences function can be employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). The BLAST sequence comparison system is available, for instance, from the NCBI web site; see also Altschul et al., J. Mol. Biol., 215:403-410, 1990; Gish. & States, Nature Genet., 3:266-272, 1993; Madden et al. Meth. Enzymol., 266:131-141, 1996; Altschul et al., Nucleic Acids Res., 25:3389-3402, 1997; and Zhang & Madden, Genome Res., 7:649-656, 1997.

In particular, the polynucleotide of the present disclosure encodes for an amino acid sequence having at least 70% of identity with SEQ ID NO:1 (i.e. a sequence having 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; or 100% of identity with SEQ ID NO:1).

As used herein, the term "polynucleotide" has its general meaning in the art and refers to a DNA or RNA molecule. However, the term captures sequences that include any of the known base analogues of DNA and RNA such as, but not limited to 4-acetylcytosine, 8-hydroxy-N6-methyladenosine, aziridinylcytosine, pseudoisocytosine, 5-(carboxyhydroxylmethyl) uracil, 5-fiuorouracil, 5-bromouracil, 5-carboxymethylaminomethyl-2-thiouracil, 5-carboxymethyl-aminomethyluracil, dihydrouracil, inosine, N6-isopentenyladenine, 1 -methyladenine, 1 -methylpseudouracil, 1-methylguanine, 1- methylinosine, 2,2-dimethylguanine, 2-methyladenine, 2-methylguanine, 3-methylcytosine, 5- methylcytosine, N6-methyladenine, 7-methylguanine, 5-methylaminomethyluracil, 5-methoxyamino-methyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, -uracil-5-oxyacetic acid methylester, uracil-5-oxyacetic acid, pseudouracil, queosine, 2-thiocytosine, and 2,6-diaminopurine.

In particular, the polynucleotide of the present disclosure comprises the nucleic acid sequence SEQ ID NO:3 (Figure 1) or a codon-optimized sequence thereof. As used herein, the term "codon optimized" means that a codon that expresses a bias for human (i.e. is common in human genes but uncommon in other mammalian genes or non-mammalian genes) is changed to a synonymous codon (a codon that codes for the same amino acid) that does not express a bias for human. Thus, the change in codon does not result in any amino acid change in the encoded protein. In particular, the polynucleotide comprises a nucleic sequence having at least 50% of identity with SEQ ID NO:3 (i.e. a sequence having 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; or 100% of identity with SEQ ID NO:3).

SEQ ID NO: 3
ATGTCTGGGGCATCTTCCTCAGAACAGAACAACAACAGCTATGAAACCAA
AACCCCAAATCTTCGAATGTCAGAGAAGAAATGTTCATGGGCCGCCTACA
TGACCAACTCCCCGACTCTGATCGTTATGATTGGTTTGCCAGCCCGGGTA
AAACCTACGTGTCCAAGAAACTAACACGCTACCTCAACTGGATTGGAGTC
CCCACCAAAGTGTTTAATCTTGGGGTGTATCGGCGTGAAGCAGTCAAGTCC
TATAAGTCCTACGACTTCTTTCGGCATGACAATGAGGAGGCCATGAAGATC
CGCAAACAGTGTGCTCTGGTGGCGCTGGAAGATGTTAAGGCGTATCTCACT
GAGGAGAATGGTCAGATTGCGGTGTTTGATGCCACCAATACAACCCGGGA
GAGGAGGGACATGATTTTGAACTTTGCTGAACAGAATTCCTTCAAGGTATT
CTTTGTGGAATCCGTCTGTGATGATCCTGATGTCATTGCTGCCAATATTCTG
GAGGTTAAGGTATCAAGCCCTGACTATCCTGAAAGGAACAGAGAGAACGT
GATGGAGGACTTCCTGAAGAGAATTGAATGCTACAAAGTTACCTACCGAC
CTCTTGACCCAGACAACTATGACAAGGATCTTTCTTTCATCAAGGTGATAA
ACGTGGGCCAGCGATTTTTAGTCAACAGAGTCCAGGACTACATCCAGAGC
AAGATAGTCTACTACCTCATGAATATCCACGTCCAGTAA

In particular, the polynucleotide of the present disclosure is included in a suitable vector.

As used herein, the term "vector" has its general meaning in the art and refers to a nucleic acid molecule into which an exogenous or heterologous or engineered nucleic acid transgene may be inserted. Vectors preferably have one or more origin of replication, and one or more site into which the recombinant DNA can be inserted. Common vectors include plasmids, viral genomes, and (primarily in yeast and bacteria) "artificial chromosomes." "Virus vectors" are defined as replication defective viruses containing the exogenous or heterologous nucleic acid transgene(s). More particularly, the vector is a viral vector. As used herein, the term "viral vector" refers to a synthetic or recombinant viral particle in which an expression cassette containing a gene of interest is packaged in a viral capsid or envelope, where any viral genomic sequences also packaged within the viral capsid or envelope are replication- deficient; i.e., they cannot generate progeny virions but retain the ability to infect target cells. In particular, the genome of the viral vector does not include genes encoding the enzymes required to replicate (the genome can be engineered to be "gutless" - containing only the transgene of interest flanked by the signals required for amplification and packaging of the artificial genome), but these genes may be supplied during production.

In particular, the viral vector is an AAV. As used herein the term "AAV" refers to the more than 30 naturally occurring and available adeno-associated viruses, as well as artificial AAVs. Typically the AAV capsid, ITRs, and other selected AAV components described herein, may be readily selected from among any AAV, including, without limitation, AAV1, AAV2, AAV3, AAV4, AAV5, AAV6, AAV6.2, AAV7, AAV8, AAV9, rh10, AAVrh64R1, AAVrh64R2, rh8, rh.10, variants of any of the known or mentioned AAVs or AAVs yet to be discovered or variants or mixtures thereof. See, e.g., WO 2005/033321. The ITRs or other AAV components may be readily isolated or engineered using techniques available to those of skill in the art from an AAV. Such AAV may be isolated, engineered, or obtained from academic, commercial, or public sources (e.g., the American Type Culture Collection, Manassas, VA). Alternatively, the AAV sequences may be engineered through synthetic or other suitable means by reference to published sequences such as are available in the literature or in databases such as, e.g., GenBank, PubMed, or the like. AAV viruses may be engineered by conventional molecular biology techniques, making it possible to optimize these particles for cell specific delivery of nucleic acid sequences, for minimizing immunogenicity, for tuning stability and particle lifetime, for efficient degradation, for accurate delivery to the nucleus, etc. As used herein, "artificial AAV" means, without limitation, an AAV with a non-naturally occurring capsid protein. Such an artificial capsid may be generated by any suitable technique, using a selected AAV sequence (e.g., a fragment of a vp1 capsid protein) in combination with heterologous sequences which may be obtained from a different selected AAV, non-contiguous portions of the same AAV, from a non-AAV viral source, or from a non-viral source. An artificial AAV may be, without limitation, a pseudotyped AAV, a chimeric AAV capsid, a recombinant AAV capsid, or a "humanized" AAV capsid. Pseudotyped vectors, wherein the capsid of one AAV is replaced with a heterologous capsid protein, are useful in the present disclosure. In particular, the AAV is AAV2/5 and AAV2/8. Methods for generating and isolating AAV viral vectors suitable for delivery to a subject are known in the art. See, e.g., US Patent 7790449; US Patent 7282199; WO 2003/042397; WO 2005/033321, WO 2006/110689; and US 7588772 B2]. In a one system, a producer cell line is transiently transfected with a construct that encodes the transgene flanked by ITRs and a construct(s) that encodes rep and cap. In a second system, a packaging cell line that stably supplies rep and cap is transiently transfected with a construct encoding the transgene flanked by ITRs. In each of these systems, AAV virions are produced in response to infection with helper adenovirus or herpesvirus, requiring the separation of the rAAVs from contaminating virus. More recently, systems have been developed that do not require infection with helper virus to recover the AAV - the required helper functions (i.e., adenovirus E1, E2a, VA, and E4 or herpesvirus UL5, UL8, UL52, and UL29, and herpesvirus polymerase) are also supplied, in trans, by the system. In these newer systems, the helper functions can be supplied by transient transfection of the cells with constructs that encode the required helper functions, or the cells can be engineered to stably contain genes encoding the helper functions, the expression of which can be controlled at the transcriptional or posttranscriptional level. In yet another system, the transgene flanked by ITRs and rep/cap genes are introduced into insect cells by infection with baculovirus-based vectors. For reviews on these production systems, see generally, e.g., Zhang et al., 2009, "Adenovirus-adeno-associated virus hybrid for large-scale recombinant adeno-associated virus production," Human Gene Therapy 20:922-929. Methods of making and using these and other AAV production systems are also described in the following U.S. patents: 5,139,941; 5,741,683; 6,057,152; 6,204,059; 6,268,213; 6,491,907; 6,660,514; 6,951,753; 7,094,604; 7,172,893; 7,201,898; 7,229,823; and 7,439,065. See generally, e.g., Grieger & Samulski, 2005, "Adeno-associated virus as a gene therapy vector: Vector development, production and clinical applications," Adv. Biochem. Engin/Biotechnol. 99: 119-145; Buning et al., 2008, "Recent developments in adeno-associated virus vector technology," J. Gene Med. 10:717-733; and the references cited below.

In particular, the vector of the present disclosure include a promoter sequence as part of the expression control sequences. In particular, a suitable promoter is a hybrid chicken β-actin (CBA) promoter with cytomegalovirus (CMV) enhancer elements. Still other suitable promoters are the CB7 promoter, as well such as viral promoters, constitutive promoters, regulatable promoters [see, e.g., WO 2011/126808 and WO 2013/04943], or a promoter responsive to physiologic cues may be used may be utilized in the in the vector. In particular, the promoter is cell-specific. The term "cell-specific" means that the particular promoter selected for the recombinant vector can direct expression of the polynucleotide of the present disclosure in a particular cell. In particular, the promoter is specific for expression of the polynucleotide in photoreceptor cells. In particular, the promoter is specific for expression in cones. Exemplary promoters may be the human G-protein-coupled receptor protein kinase 1 (GRK1) promoter (Genbank Accession number AY327580). In particular, the promoter is a 292 nt fragment (positions 1793-2087) of the GRK1 promoter (See, Beltran et al, Gene Therapy 2010 17:1162-74). In particular, the promoter is the human interphotoreceptor retinoid-binding protein proximal (IRBP) promoter. In particular, the promoter is a 235 nt fragment of the hIRBP promoter. In particular, the promoter is the RPGR proximal promoter (Shu et al, IOVS, May 2102). Other promoters useful in the present disclosure include, without limitation, the rod opsin promoter, the red-green opsin promoter, the blue opsin promoter, the cGMP-β-phosphodiesterase promoter, the mouse opsin promoter (Beltran et al 2010 cited above), the rhodopsin promoter (Mussolino et al, Gene Ther, July 2011, 18(7):637-45); the alpha-subunit of cone transducin (Morrissey et al, BMC Dev, Biol, Jan 2011, 11:3); beta phosphodiesterase (PDE) promoter; the retinitis pigmentosa (RP1) promoter (Nicord et al, J. Gene Med, Dec 2007, 9(12):1015-23); the NXNL2/NXNL1 promoter (Lambard et al, PLoS One, Oct. 2010, 5(10):e13025), the retinal degeneration slow/peripherin 2 (Rds/perph2) promoter (Cai et al, Exp Eye Res. 2010 Aug;91(2):186-94); and the VMD2 promoter (Kachi et al, Human Gene Therapy, 2009 (20:31-9)).

As used herein the term "administering" means delivering the polynucleotide to the target selected cells, in particular cones. Typical routes of administration typically include systemic routes, e.g., intraarterial, intraocular, intravenous, intramuscular, subcutaneous, intradermal, and other parental routes of administration. Direct delivery to the eye optionally via ocular delivery, intra-retinal injection, intravitreal, topical represent a particular interest for the treatment of the retinal degenerative diseases. Routes of administration may be combined, if desired. In particular, the administration is repeated periodically. The polynucleotide of the present disclosure may be delivered in a single composition or multiple compositions. Optionally, two or more different AAV may be delivered, or multiple viruses [see, e.g., WO20 2011/126808 and WO 2013/049493]. In particular, multiple viruses may contain different replication-defective viruses (e.g., AAV and adenovirus), alone or in combination with proteins.

By a "therapeutically effective amount" is meant a sufficient amount of the polynucleotide of the present disclosure for the treatment of the retinal degenerative disease at a reasonable benefit/risk ratio applicable to any medical treatment. It will be understood that the total daily usage of the compounds and compositions of the present disclosure will be decided by the attending physician within the scope of sound medical judgment. The specific therapeutically effective dose level for any particular subject will depend upon a variety of factors including the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

According to the present disclosure, the polynucleotide of the present disclosure typically inserted in a viral vector is administered to the subject in the form of a pharmaceutical composition. Typically, the polynucleotide of the present disclosure may be combined with pharmaceutically acceptable excipients, and optionally sustained-release matrices, such as biodegradable polymers, to form pharmaceutical compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present disclosure for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the present disclosure as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The polynucleotide of the present disclosure can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the typical methods of preparation are vacuum-drying and freeze-drying techniques which yield a powder of the active ingredient plus any additional desired ingredient from a previously sterile-filtered solution thereof. The preparation of more, or highly concentrated solutions for direct injection is also contemplated, where the use of DMSO as solvent is envisioned to result in extremely rapid penetration, delivering high concentrations of the active agents to a small tumor area. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

The present disclosure will be further illustrated by the following figures and examples. However, these examples and figures should not be interpreted in any way as limiting the scope of the present disclosure.

### FIGURES:

**Figure 1****: sequence of PFKFB2 kinase domain.**
**Figure 2****: PFKFB2 is expressed by cones in a rod-dependant manner. A.** Expression of PFKFB2 in the heart, brain and retina on the wild-type mouse by western blotting. **B.** Differential expression of PFKFB2 in the wild-type and *rd1* retina at post natal day 21 and 35 by western blotting. **C.** quantification of the signal. **D.** Expression of *Rho* in the wild-type and *rd1* by microarray analysis. By postnatal day 21, the majority of rods have degenerated.
**Figure 3****:A.** The expression of *Pfkfb2* is induced by glucose in the cone-enriched cultures from chicken embryo. **B.** Electroporation of mouse *Pjkfb2* cDNA plasmid increases cone survival in the cone-enriched cultures from chicken embryo.

### EXAMPLE:

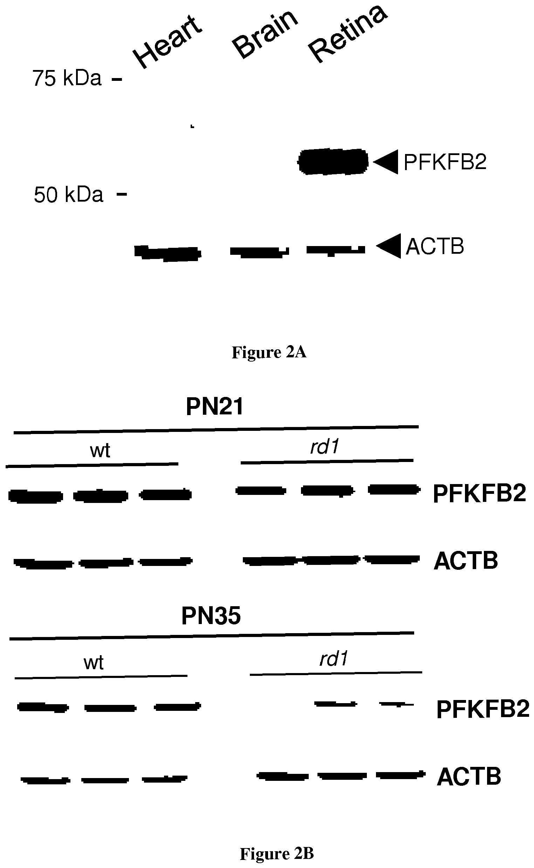

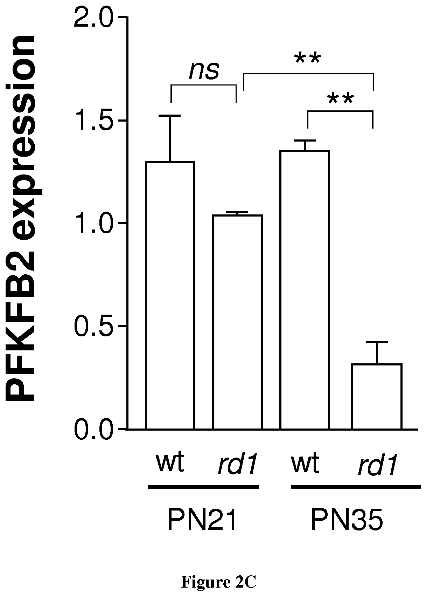

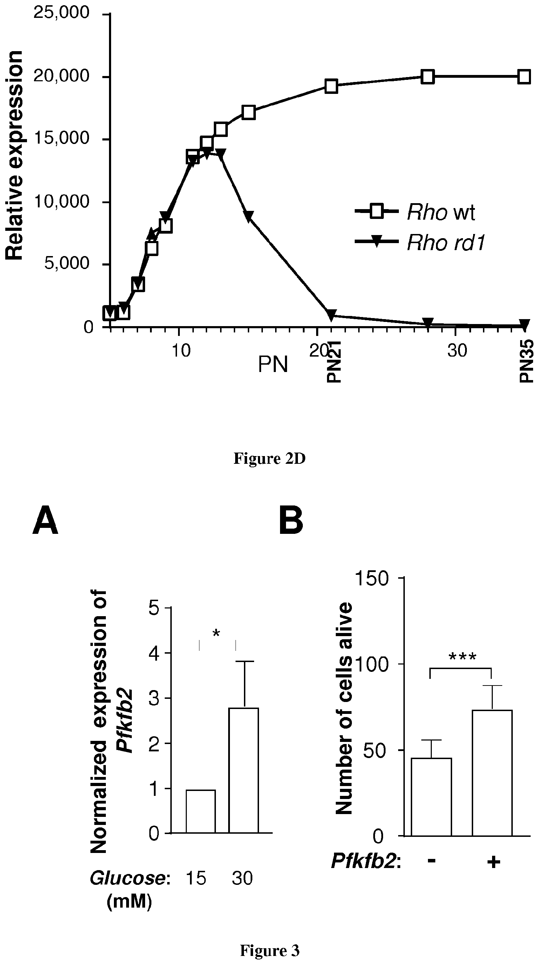

In the prior art, PFKFB2 is described as being expressed mainly in the heart. However we showed unexpectedly that this protein is expressed in high amount in retina in comparison with heart (Figure 2A). We further showed that PFKFB2 is expressed by cones in a rod-dependant manner. In particular, we showed that its expression follows the viability of cones (Figures 2B-D): its expression is lost a day 35 in an animal model retinitis pigmentosa (*rd1* mouse). The expression of PFKFB2 is induced by glucose (Figure 3A) and we accumulate evidences that this protein, especially its kinase domain, is involved in the mechanism of action of RdCVF. More particularly we showed that transduction of a polynucleotide encoding for PFKFB2 increases cone survival (Figure 3A). We now perform experiments in the *rd1* animal model with AAV (AAV2.8) vectors comprising the polynucleotide encoding for the PFKFB2 kinase domain.

### REFERENCES:

Throughout this application, various references describe the state of the art to which this present disclosure pertains.
Aït-Ali N, Fridlich R, Millet-Puel G, Clérin E, Delalande F, Jaillard C, Blond F, Perrocheau L, Reichman S, Byrne LC, Olivier-Bandini A, Bellalou J, Moyse E, Bouillaud F, Nicol X, Dalkara D, van Dorsselaer A, Sahel JA, Léveillard T. Rod-derived cone viability factor promotes cone survival by stimulating aerobic glycolysis. Cell. 2015 May 7;161(4):817-32.
T. Léveillard, S. Mohand-Saïd, O. Lorentz, D. Hicks, A.C. Fintz, E. Clérin, M. Simonutti, V. Forster, N. Cavusoglu, F. Chalmel, et al. Identification and characterization of rod-derived cone viability factor Nat. Genet., 36 (2004), pp. 755-759
G. Elachouri, I. Lee-Rivera, E. Clerin, M. Argentini, R. Fridlich, F. Blond, V. Ferracane, Y. Yang, W. Raffelsberger, J. Wan, et al. The thioredoxin RdCVFL protects against photooxidative retinal damage Free Radic. Biol. Med., 81 (2015), pp. 22-29.
R. Fridlich, F. Delalande, C. Jaillard, J. Lu, L. Poidevin, T. Cronin, L. Perrocheau, G. Millet-Puel, M.L. Niepon, O. Poch, et al. The thioredoxin-like protein rod-derived cone viability factor (RdCVFL) interacts with TAU and inhibits its phosphorylation in the retina Mol. Cell. Proteomics, 8 (2009), pp. 1206-1218.
L.C. Byrne, D. Dalkara, G. Luna, S.K. Fisher, E. Clérin, J.A. Sahel, T. Léveillard, J.G. Flannery Viral-mediated RdCVF and RdCVFL expression protects cone and rod photoreceptors in retinal degeneration J. Clin. Invest., 125 (2015), pp. 105-116
Y. Yang, S. Mohand-Said, A. Danan, M. Simonutti, V. Fontaine, E. Clerin, S. Picaud, T. Leveillard, J.A. Sahel Functional cone rescue by RdCVF protein in a dominant model of retinitis pigmentosa Mol. Ther., 17 (2009), pp. 787-795.

## Claims

1. A polynucleotide encoding for the PFKFB2 kinase domain for use in the treatment of a retinal degenerative disease in a subject in need thereof.

2. The polynucleotide encoding for the PFKFB2 kinase domain for use according to claim 1 wherein the retinal degenerative disease is a cone dystrophy.

3. The polynucleotide encoding for the PFKFB2 kinase domain for use according to claim 1 wherein the retinal degenerative disease is selected from the group consisting of retinitis pigmentosa (RP), Leber congenital amaurosis (LCA), age-related macular degeneration (AMD), recessive RP, dominant RP, X-linked RP, incomplete X-linked RP, dominant, dominant LCA, recessive ataxia, posterior column with RP, recessive RP with para-arteriolar preservation of the RPE, RP 12, Usher syndrome, dominant retinitis pigmentosa with sensorineural deafness, recessive retinitis punctata albescens, recessive Alstrδm syndrome, recessive Bardet-Biedl syndrome, dominant spinocerebellar ataxia w/ macular dystrophy or retinal degeneration, Recessive abetalipoproteinemia, recessive retinitis pigmentosa with macular degeneration, recessive Refsum disease adult form, recessive Refsum disease infantile form, recessive enhanced S-cone syndrome, RP with mental retardation, RP with myopathy, recessive Newfoundland rod-cone dystrophy, RetRP sinpigmento, sector RP, regional RP, Senior-Loken syndrome, Joubert syndrome, Stargardt disease juvenile, Stargardt disease late onset, dominant macular dystrophy Stargardt type, dominant Stargardt-like macular dystrophy, recessive macular dystrophy, recessive fundus flavimaculatus, recessive cone-rod dystrophy, X- linked progressive cone-rod dystrophy, dominant cone-rod dystrophy, cone-rod dystrophy; de Grouchy syndrome, dominant cone dystrophy, X-linked cone dystrophy, recessive cone dystrophy, recessive cone dystrophy with supernormal rod electroretinogram, X-linked atrophic macular dystrophy, X-linked retinoschisis, dominant macular dystrophy, dominant radial, macular drusen, dominant macular dystrophy, bull's-eye, dominant macular dystrophy butterfly-shaped, dominant adult vitelliform macular dystrophy, dominant macular dystrophy North Carolina type, dominant retinal-cone dystrophy 1, dominant macular dystrophy cystoid, dominant macular dystrophy, atypical vitelliform, foveomacular atrophy, dominant macular dystrophy Best type, dominant macular dystrophy North Carolina-like with progressive, recessive macular dystrophy juvenile with hypotrichosis, recessive foveal hypoplasia and anterior segment dysgenesis, recessive delayed cone adaptation, macular dystrophy in blue cone monochromacy, macular pattern dystrophy with type II diabetes and deafness, Flecked retina of Kandori, pattern dystrophy, dominant Stickler syndrome, dominant Marshall syndrome, dominant vitreoretinal degeneration, dominant familial exudative vitreoretinopathy, dominant vitreoretinochoroidopathy; dominant neovascular inflammatory vitreoretinopathy, Goldmann-Favre syndrome, recessive achromatopsia, dominant tritanopia, recessive rod monochromacy, congenital red-green deficiency, deuteranopia, protanopia, deuteranomaly, protanomaly, recessive Oguchi disease, dominant macular dystrophy late onset, recessive gyrate atrophy, dominant atrophia areata, dominant central areolar choroidal dystrophy, X-linked choroideremia, choroidal atrophy, central areolar, central, peripapillary, dominant progressive bifocal chorioretinal atrophy, progresive bifocal choroioretinal atrophy, dominant Doyne honeycomb retinal degeneration (Malattia Leventinese), amelogenesis imperfecta, recessive Bietti crystalline corneoretinal dystrophy, dominant hereditary vascular retinopathy with Raynaud phenomenon and migraine, dominant Wagner disease and erosive vitreoretinopathy, recessive microphthalmos and retinal disease syndrome; recessive nanophthalmos, recessive retardation, spasticity and retinal degeneration, recessive Bothnia dystrophy, recessive pseudoxanthoma elasticum, dominant pseudoxanthoma elasticum; recessive Batten disease (ceroid-lipofuscinosis), juvenile, dominant Alagille syndrome, McKusick- Kaufman syndrome, hypoprebetalipoproteinemia, acanthocytosis, palladial degeneration; Recessive Hallervorden-Spatz syndrome; dominant Sorsby's fundus dystrophy, Oregon eye disease, Kearns-Sayre syndrome, RP with developmental and neurological abnormalities, Basseb Korenzweig Syndrome, Hurler disease, Sanfilippo disease, Scieie disease, melanoma associated retinopathy, Sheen retinal dystrophy, Duchenne macular dystrophy, Becker macular dystrophy, Birdshot Retinochoroidopathy, multiple evanescent white-dot syndrome, acute zonal occult outer retinopathy, retinal vein occlusion, retinal artery occlusion, diabetic retinopathy, retinal toxicity, retinal injury, retinal traumata and retinal laser lesions, and Fundus Albipunctata, retinal detachment, diabetic retinopathy, and retinopathy of prematurity.

4. The polynucleotide encoding for the PFKFB2 kinase domain for use according to claim 1 wherein the retinal degenerative disease is retinitis pigmentosa.

5. The polynucleotide encoding for the PFKFB2 kinase domain for use according to claim 1 wherein the polynucleotide encodes for an amino acid sequence having at least 80% of identity with SEQ ID NO:2.

6. The polynucleotide encoding for the PFKFB2 kinase domain for use according to claim 1 wherein the polynucleotide encodes for an amino acid sequence having at least 70% of identity with SEQ ID NO:1.

7. The polynucleotide encoding for the PFKFB2 kinase domain for use according to claim 1 wherein the polynucleotide comprises the nucleic acid sequence SEQ ID NO:3 or a codon-optimized sequence thereof.

8. The polynucleotide encoding for the PFKFB2 kinase domain for use according to claim 1 wherein the polynucleotide comprises a nucleic sequence having at least 50% of identity with SEQ ID NO:3.

9. The polynucleotide encoding for the PFKFB2 kinase domain for use according to claim 1 wherein the polynucleotide is included in a suitable vector.

10. The polynucleotide encoding for the PFKFB2 kinase domain for use according to claim 9 wherein the vector is a viral vector such as an AVV.

11. The polynucleotide encoding for the PFKFB2 kinase domain for use according to claim 10 wherein the AAV is AAV2/5 and AAV2/8.

12. The polynucleotide encoding for the PFKFB2 kinase domain for use according to claim 9 wherein the vector includes a promoter sequence as part of the expression control sequences.

13. The polynucleotide encoding for the PFKFB2 kinase domain for use according to claim 12 wherein the promoter is specific for expression in cones.

14. The polynucleotide encoding for the PFKFB2 kinase domain for use according to claim 13 wherein the polynucleotide is delivered to the eye optionally via ocular, intra-retinal injection, intravitreal, or topical delivery.

## Patentansprüche

1. Polynukleotid, das für die PFKFB2-Kinasedomäne codiert, zur Verwendung bei der Behandlung einer netzhautdegenerativen Erkrankung bei einem Subjekt mit Bedarf daran.

2. Polynukleotid, das für die PFKFB2-Kinasedomäne codiert, zur Verwendung nach Anspruch 1, wobei die netzhautdegenerative Erkrankung eine Zapfendystrophie ist.

3. Polynukleotid, das für die PFKFB2-Kinasedomäne codiert, zur Verwendung nach Anspruch 1, wobei die netzhautdegenerative Erkrankung ausgewählt ist aus der Gruppe bestehend aus Retinitis pigmentosa (RP), Leberscher kongenitaler Amaurose (LCA), alterbedingter Makuladegeneration (AMD), rezessiver RP, dominanter RP, X-gebundener RP, unvollständiger X-gebundener RP, dominanter, dominanter LCA, rezessiver Ataxie, hinterer Säule mit RP, rezessiver RP mit para-arteriolärer Erhaltung der RPE, RP 12, Usher-Syndrom, dominanter Retinitis pigmentosa mit sensorineuraler Taubheit, rezessiver Retinitis punctata albescens, rezessivem Alström-Syndrom, rezessivem Bardet-Biedl-Syndrom, dominanter spinozerebellarer Ataxie mit Makuladystrophie oder Netzhautdegeneration, rezessiver Abetalipoproteinämie, rezessiver Retinitis pigmentosa mit Makuladegeneration, rezessivem Refsum-Syndrom bei Erwachsenen, rezessivem Refsum-Syndrom bei Kindern, rezessivem verstärkten Blauzapfensyndrom, RP mit geistiger Retardierung, RP mit Myopathie, rezessiver Neufundland Stäbchen-Zapfendystrophie, RetRP sine pigmento, Sektor-RP, regionaler RP, Senior-Loken-Syndrom, Joubert-Syndrom, Morbus Stargardt bei Jugendlichen, spätmanifestem Morbus Stargardt, dominanter Makuladystrophie vom Stargardt-Typ, dominanter Stargardt-artiger Makuladystrophie, rezessiver Makuladystrophie, rezessivem Fundus flavimaculatus, rezessiver Zapfen-Stäbchen-Dystrophie, X-gebundener progressiver Zapfen-Stäbchen-Dystrophie, dominanter Zapfen-Stäbchen-Dystrophie, Zapfen-Stäbchen-Dystrophie; De Grouchy-Syndrom, dominanter Zapfendystrophie, X-gebundener Zapfendystrophie, rezessiver Zapfendystrophie, rezessiver Zapfendystrophie mit supernormalem Stäbchen-Elektroretinogramm, X-gebundener atrophischer Makuladystrophie, X-gebundener Retinoschisis, dominanter Makuladystrophie, dominanten radialen Maculadrusen, dominanter Makuladystrophie, Bulls-Eye, dominanter schmetterlingsförmiger Makuladystrophie, dominanter adulter vitelliformer Makuladystrophie, dominanter Makuladystrophie vom North Carolina-Typ, dominanter retinaler Zapfendystrophie 1, dominanter zystoider Makuladystrophie, dominanter Makuladystrophie, atypischer vitelliformer, fovealer Makulaatrophie, dominanter Makuladystrophie vom Best-Typ, dominanter Makuladystrophie North Carolina-artig mit progressiver, rezessiver, juveniler Makuladystrophie mit Hypotrichose, rezessiver fovealer Hypoplasie und Dysgenese des vorderen Segments, rezessiver verzögerter Zapfenanpassung, Makuladystrophie in Blauzapfenmonochromasie, Makulamusterdystrophie mit Diabetes Typ II und Taubheit, Kandoris gefleckter Retina, Musterdystrophie, dominantem Stickler-Syndrom, dominantem Marshall-Syndrom, dominanter vitreoretinaler Degeneration, dominanter familiärer exudativer Vitreoretinopathie, dominanter Vitreoretinochoroidopathie; dominanter neovaskulärer entzündlicher Vitreoretinopathie, Goldmann-Favre-Syndrom, rezessiver Achromatopsie, dominanter Tritanopie, rezessiver Stäbchenmonochromasie, kongenitaler Rot-Grün-Blindheit, Deuteranopie, Protanopie, Deuteranomalie, Protanomalie, rezessivem Oguchi-Syndrom, dominanter spätmanifester Makuladystrophie, rezessiver Atrophia gyrata, dominanter Atrophia gyrata, dominanter zentraler areolärer choroidaler Dystrophie, X-gebundener Chorioideremie, choroidaler Atrophie, zentraler areolärer, zentraler, peripapillärer, dominanter progressiver bifokaler chorioretinaler Atrophie, progressiver bifokaler chorioretinaler Atrophie, dominanter Doyne-Honeycomb-Netzhautdegeneration (Malattia Leventinese), Amelogenesis imperfecta, rezessiver comeoretinaler Bietti-Kristalldystrophie, dominanter erbbedingter vaskulärer Retinopathie mit Raynaud-Phänomen und Migräne, dominanter Wagner-Krankheit und erosiver Vitreoretinopathie, rezessivem Mikrophthalmus und Netzhauterkrankungssyndrom; rezessivem Nanophthalmus, rezessiver Retardation, Spastik und Netzhautdegeneration, rezessiver Bothnia-Dystrophie, rezessivem Pseudoxanthoma elasticum, dominantem Pseudoxanthoma elasticum; rezessiver Batten-Krankheit (Ceroidlipofuszinose), juvenilem dominanten Alagille-Syndrom, McKusick-Kaufman-Syndrom, Hypoprebetalipoproteinämie, Akanthozytose, Palladiumdegeneration; rezessivem Hallervorden-Spatz-Syndrom; dominanter Sorsby-Dystrophie, Oregon-Augenkrankheit, Kearns-Sayre-Syndrom, RP mit Entwicklungs- und neurologischen Anomalien, Bassen-Kornzweig-Syndrom, Morbus Hurler, Sanfilippo-Syndrom, Scieie-Krankheit, melanomassoziierter Retinopathie, Sheen-Netzhautdystrophie, Duchenne-Makuladystrophie, Becker-Makuladystrophie, Birdshot-Retinochoroidopathie, multiplem evaneszenten White-Dot-Syndrom, akuter zonaler okkulter äußerer Retinopathie, Netzhautvenenthrombose, Netzhautarterienthrombose, diabetischer Retinopathie, Netzhauttoxizität, Netzhautverletzung, Netzhauttraumata und Netzhautlaserläsionen und Fundus Albipunctata, Netzhautablösung, diabetischer Retinopathie und Frühgeborenen-Retinopathie.

4. Polynukleotid, das für die PFKFB2-Kinasedomäne codiert, zur Verwendung nach Anspruch 1, wobei die netzhautdegenerative Erkrankung Retinitis pigmentosa ist.

5. Polynukleotid, das für die PFKFB2-Kinasedomäne codiert, zur Verwendung nach Anspruch 1, wobei das Polynukleotid für eine Aminosäuresequenz mit mindestens 80% Identität mit SEQ ID NR:2 codiert.

6. Polynukleotid, das für die PFKFB2-Kinasedomäne codiert, zur Verwendung nach Anspruch 1, wobei das Polynukleotid für eine Aminosäuresequenz mit mindestens 70% Identität mit SEQ ID NR: 1 codiert.

7. Polynukleotid, das für die PFKFB2-Kinasedomäne codiert, zur Verwendung nach Anspruch 1, wobei das Polynukleotid die Nukleinsäuresequenz SEQ ID NR:3 oder eine kodonoptimierte Sequenz davon umfasst.

8. Polynukleotid, das für die PFKFB2-Kinasedomäne codiert, zur Verwendung nach Anspruch 1, wobei das Polynukleotid eine Nukleinsäuressequenz mit mindestens 50% Identität mit SEQ ID NR:3 umfasst.

9. Polynukleotid, das für die PFKFB2-Kinasedomäne codiert, zur Verwendung nach Anspruch 1, wobei das Polynukleotid in einem geeigneten Vektor beinhaltet ist.

10. Polynukleotid, das für die PFKFB2-Kinasedomäne codiert, zur Verwendung nach Anspruch 9, wobei der Vektor ein viraler Vektor wie ein AVV ist.

11. Polynukleotid, das für die PFKFB2-Kinasedomäne codiert, zur Verwendung nach Anspruch 10, wobei der AAV AAV2/5 und AAV2/8 ist.

12. Polynukleotid, das für die PFKFB2-Kinasedomäne codiert, zur Verwendung nach Anspruch 9, wobei der Vektor eine Promotorsequenz als Teil der Expressionssteuersequenzen beinhaltet.

13. Polynukleotid, das für die PFKFB2-Kinasedomäne codiert, zur Verwendung nach Anspruch 12, wobei der Promotor für Expression in Zapfen spezifisch ist.

14. Polynukleotid, das für die PFKFB2-Kinasedomäne codiert, zur Verwendung nach Anspruch 13, wobei das Polynukleotid an das Auge optional über okuläre, intra-retinale Injektion, intravitreale oder topische Abgabe abgegeben wird.

## Revendications

1. Polynucléotide codant pour le domaine kinase de la PFKFB2 destiné à être utilisé dans le traitement d'une maladie dégénérative de la rétine chez un sujet qui en a besoin.

2. Polynucléotide codant pour le domaine kinase de la PFKFB2 destiné à être utilisé selon la revendication 1 dans lequel la maladie dégénérative de la rétine est une dystrophie des cônes.

3. Polynucléotide codant pour le domaine kinase de la PFKFB2 destiné à être utilisé selon la revendication 1 dans lequel la maladie dégénérative de la rétine est choisie parmi le groupe consistant en rétinite pigmentaire (RP), amaurose congénitale de Leber (ACL), dégénérescence maculaire liée à l'âge (DMLA), RP récessive, RP dominante, RP liée à l'X, RP incomplète liée à l'X, dominante, ACL dominante, ataxie récessive, colonne postérieure avec RP, RP récessive avec préservation para-artériolaire de l'EPR, RP 12, syndrome d'Usher, rétinite pigmentaire dominante avec surdité neurosensorielle, rétinite ponctuée albescente récessive, syndrome d'Alström récessif, syndrome de Bardet Biedl récessif, ataxie spinocérébelleuse dominante avec dystrophie maculaire ou dégénérescence rétinienne, abêtalipoprotéinémie récessive, rétinite pigmentaire récessive avec dégénérescence maculaire, maladie de Refsum de l'adulte récessive, maladie de Refsum infantile récessive, syndrome du cône S amélioré récessif, RP avec retard mental, RP avec myopathie, dystrophie de type cônes-bâtonnets de Terre-Neuve récessive, rétinite pigmentaire sine pigmento, RP en secteur, RP en région, syndrome de Senior-Loken, syndrome de Joubert, maladie de Stargardt juvénile, maladie de Stargardt à révélation tardive, forme dominante de la dystrophie maculaire de Stargardt, dystrophie maculaire dominante de Stargardt, dystrophie maculaire récessive, fundus flavimaculatus récessif, dystrophie cônes-bâtonnets récessive, dystrophie cônes-bâtonnets progressive liée à l'X, dystrophie cônes-bâtonnets dominante, dystrophie cônes-bâtonnets ; syndrome de de Grouchy, dystrophie des cônes dominante, dystrophie des cônes liée à l'X, dystrophie des cônes récessive, dystrophie des cônes récessive avec électrorétinogramme des bâtonnets supernormal, dystrophie maculaire atrophique liée à l'X, rétinoschisis lié à l'X, dystrophie maculaire dominante, radiale dominante, drusen maculaire, dystrophie maculaire dominante, en oeil de boeuf, dystrophie maculaire dominante en forme de papillon, dystrophie maculaire vitelliforme dominante de l'adulte, dystrophie maculaire dominante de la Caroline du Nord, dystrophie dominante du cône rétinien 1, dystrophie maculaire cystoïde dominante, dystrophie maculaire dominante, vitelliforme atypique, atrophie fovéo-maculaire, dystrophie maculaire dominante de Best, dystrophie maculaire dominante de la Caroline du Nord avec dystrophie maculaire progressive et récessive juvénile avec hypotrichose, hypoplasie fovéale récessive et dysgénésie du segment antérieur, adaptation récessive retardée des cônes, dystrophie maculaire avec monochromatisme à cônes bleus, dystrophie maculaire avec diabète de type II et surdité, syndrome de Kandori, dystrophie en pattern, syndrome de Stickler dominant, syndrome de Marshall dominant, dégénérescence vitréorétinienne dominante, vitréorétinopathie exsudative familiale dominante, vitréo-rétino-choroïdopathie dominante ; vitréorétinopathie inflammatoire néovasculaire dominante, syndrome de Goldmann-Favre, achromatopsie récessive, tritanopie dominante, monochromatisme récessive à bâtonnets, déficience rouge-vert congénitale, deutéranopie, protanopie, deutéranomalie, protanomalie, maladie d'Oguchi récessive, dystrophie maculaire dominante à révélation tardive, atrophie gyrée récessive, atrophie areata dominante, dystrophie choroïdienne aréolaire centrale dominante, choroïdérémie liée à l'X, atrophie choroïdienne, aréolaire centrale, centrale, péripapillaire, atrophie choriorétinienne bifocale progressive dominante, atrophie choriorétinienne bifocale progressive, dégénérescence rétinienne en rayon de miel de Doyne dominante (Malattia Leventinese), amélogenèse imparfaite, dystrophie choriorétinienne cristalline de Bietti récessive , rétinopathie vasculaire héréditaire dominante avec phénomène de Raynaud et migraine, maladie de Wagner dominante et vitréorétinopathie érosive, microphtalmie récessive et syndrome de maladie rétinienne ; nanophtalmie récessive, à retard récessive, spasticité et dégénérescence rétinienne, dystrophie rétinienne type Botnie récessive, pseudoxanthome élastique récessif, pseudoxanthome élastique dominant; maladie de Batten récessive (céroïde-lipofuscinose), juvénile, syndrome d'Alagille dominant, syndrome de McKusick Kaufman, hypobêtalipoprotéinémie, acanthocytose, dégénérescence palladienne, Syndrome d'Hallervorden-Spatz, dystrophie du fond d'œil de Sorsby dominante, maladie oculaire d'Oregon, syndrome de Kearns-Sayre, RP avec anomalies développementales et neurologiques, syndrome de Bassen-Kornzweig, maladie de Hurler, maladie de Sanfilippo, maladie de Scieie, rétinopathie associée au mélanome, dystrophie rétinienne de Sheen, dystrophie maculaire de Duchenne, dystrophie maculaire de Becker, rétinochoroïdopathie de type birdshot, syndrome des taches blanches évanescentes et multiples, rétinopathie externe occulte zonale aiguë, occlusion veineuse rétinienne, occlusion de l'artère rétinienne, rétinopathie diabétique, toxicité rétinienne, lésion rétinienne, traumatismes rétiniens et lésions rétiniennes au laser, et Fundus Albipunctatus, décollement de la rétine, rétinopathie diabétique et rétinopathie du prématuré.

4. Polynucléotide codant pour le domaine kinase de la PFKFB2 destiné à être utilisé selon la revendication 1 dans lequel la maladie dégénérative de la rétine est une rétinite pigmentaire.

5. Polynucléotide codant pour le domaine kinase de la PFKFB2 destiné à être utilisé selon la revendication 1 dans lequel le polynucléotide code pour une séquence d'acides aminés présentant une identité d'au moins 80 % avec la SEQ ID NO : 2.

6. Polynucléotide codant pour le domaine kinase de la PFKFB2 destiné à être utilisé selon la revendication 1 dans lequel le polynucléotide code pour une séquence d'acides aminés présentant une identité d'au moins 70 % avec la SEQ ID NO : 1.

7. Polynucléotide codant pour le domaine kinase de la PFKFB2 destiné à être utilisé selon la revendication 1 dans lequel le polynucléotide comprend la séquence d'acides nucléiques SEQ ID NO : 3 ou une séquence à codons optimisés de celle-ci.

8. Polynucléotide codant pour le domaine kinase de la PFKFB2 destiné à être utilisé selon la revendication 1 dans lequel le polynucléotide comprend une séquence d'acides nucléiques présentant une identité d'au moins 50 % avec la SEQ ID NO : 3.

9. Polynucléotide codant pour le domaine kinase de la PFKFB2 destiné à être utilisé selon la revendication 1 dans lequel le polynucléotide est inclus dans un vecteur approprié.

10. Polynucléotide codant pour le domaine kinase de la PFKFB2 destiné à être utilisé selon la revendication 9 dans lequel le vecteur est un vecteur viral tel qu'un AVV.

11. Polynucléotide codant pour le domaine kinase de la PFKFB2 destiné à être utilisé selon la revendication 10 dans lequel l'AVV est AVV2/5 et AVV2/8.

12. Polynucléotide codant pour le domaine kinase de la PFKFB2 destiné à être utilisé selon la revendication 9 dans lequel le vecteur inclut une séquence promotrice dans le cadre des séquences de régulation de l'expression.

13. Polynucléotide codant pour le domaine kinase de la PFKFB2 destiné à être utilisé selon la revendication 12 dans lequel le promoteur est spécifique à l'expression dans les cônes.

14. Polynucléotide codant pour le domaine kinase de la PFKFB2 destiné à être utilisé selon la revendication 13 dans lequel le polynucléotide est administré dans l'œil facultativement par une administration par voie oculaire, par injection intra-rétinienne, une administration intra-vitréenne ou topique.
